Europäisches Patentamt

European Patent Office

Office européen des brevets

(19)

(11) **EP 0 710 833 A2**

(12) **EUROPÄISCHE PATENTANMELDUNG**

(43) Veröffentlichungstag:
08.05.1996 Patentblatt 1996/19

(51) Int. Cl.[6]: **G01N 21/77**

(21) Anmeldenummer: 95117289.9

(22) Anmeldetag: 02.11.1995

(84) Benannte Vertragsstaaten:
AT CH DE ES FR GB IT LI

(30) Priorität: 04.11.1994 DE 4439348

(71) Anmelder: BOEHRINGER MANNHEIM GMBH
D-68305 Mannheim-Waldhof (DE)

(72) Erfinder:
• Weindel, Kurt, Dr.
D-82392 Habach (DE)

• Hornauer, Hans, Dr.
D-82392 Habach (DE)

(74) Vertreter: Weiss, Wolfgang, Dr. Dipl.-Chem. et al
Patentanwälte
Weickmann & Partner,
Kopernikusstrasse 9
D-81679 München (DE)

(54) **Weisse Trigger-Präparationen zur Verbesserung der Signaldetektion bei bio- und chemilumineszenten Reaktionen**

(57)    Die vorliegende Erfindung betrifft ein Verfahren zum Nachweis eines Analyten in einer Probeflüssigkeit durch Lumineszenzmessung nach dem Prinzip des Ligand-Rezeptor-Assay, z.B. eines Immunoassay oder eines Hybridisierungsassay oder einer Kombination davon, wobei man eine Probeflüssigkeit mit mindestens einem Rezeptor inkubiert, der eine Lumineszenzmarkierung trägt, und das Vorhandensein oder/und die Menge des nachzuweisenden Analyten in der Probeflüssigkeit durch Lumineszenzmessung in einem Meßmedium mit dispergierten Komponenten bestimmt.

EP 0 710 833 A2

**Beschreibung**

Die vorliegende Erfindung betrifft ein Verfahren zum Nachweis eines Analyten in einer Probeflüssigkeit durch Lumineszenzmessung nach dem Prinzip des Ligand-Rezeptor-Assay, z.B. eines Immunoassay oder eines Hybridisierungsassay oder einer Kombination davon, wobei man eine Probeflüssigkeit mit mindestens einem Rezeptor inkubiert, der eine Lumineszenzmarkierung trägt, und das Vorhandensein oder/und die Menge des nachzuweisenden Analyten in der Probeflüssigkeit durch Lumineszenzmessung bestimmt.

Die Verwendung von Lumineszenz-Direktmarkierungen, wie z.B. Isoluminol oder Acridiniumverbindungen, oder enzymverstärkten Lumineszenzmarkierungen, wie z.B. Meerrettich-Peroxidase/Luminol oder alkalische Phosphatase/stabilisierte Dioxetane als Nachweissystem bei immunologischen Testverfahren hat gegenüber anderen Nachweissystemen, z.B. radioaktiven Markierungsgruppen, den Vorteil einer höheren Sensitivität (siehe u.a. McCapra F. und Beheshti I. in Knox van Dyke (Hg.): Bioluminescence and Chemiluminescence; Instruments and Applications, Vol. 1, 9-42, 1985; Barnard G.J.R. et al. in Knox van Dyke (Hg.): Bioluminescence and Chemiluminescence; Instruments and Applications, Vol. 1, 151-183, 1985; Weeks I. et al. in de Luca M.A. und McElroy W.D. (Hgs.): Methods in Enzymology, Vol. 133, 366-387, 1986; McCapra F., et al., Journal of Bioluminescence and Chemiluminescence 4, 51-58, 1989; Bronstein I. und McGrath P., Nature 338, 599-600, 1989; Thorpe G.H. und Kricka L.J., Journal of Bioluminescence and Chemiluminescence 3, 97-100, 1989).

Ein Nachteil von Lumineszenzmarkierungen besteht jedoch in der geringen Signalstärke. Diese ist darauf zurückzuführen, daß bei der Lumineszenzmessung keine Signalmultiplikation möglich ist, d.h. jede Direktmarkierung bzw. jedes lumineszente Enzymsubstratmolekül wird in einer einzigen lichterzeugenden Reaktion erschöpft. Daher bestehen seit langem Bestrebungen, diesen Nachteil zu beseitigen.

So ist beispielsweise eine Verstärkung der Chemilumineszenz aus der Peroxidase (POD)-katalysierten Luminol-Oxidation durch Benzothiazole (Whitehead T.P. et al., Nature 305, 158-159, 1983), p-substituierte Phenole wie z.B. p-Iodphenol (Thorpe G.H. et al., Clinical Chemistry 31, 1335-1341, 1985; Coyle P.M. et al., Annals of Clinical Biochemistry 23, 42-46, 1986), Fluorescein (EP 0 228 046 B1), o- oder p-Thiazolyl- bzw. o- oder p-Thienylphenole (EP 0 455 471 A2), Hydroxyfluorenone (W0 90/13665) bekannt. Diese Verstärkungsmoleküle bewirken eine bessere Koordination der lichterzeugenden Luminol-Oxidation.

Weiterhin ist eine Verstärkung der alkalischen Phosphatasekatalysierten Chemilumineszenz von stabilisierten, triggerbaren Dioxetanen durch Polyvinylbenzyl(benzyldimethylammonium)chloride (US-Patent 5 145 772 von Bronstein I. et al.; Tropix News Letter on Chemiluminescent Substrates, 1993), Polyvinylbenzyl-trialkyl-phosphonium-Salze (EP 0 561 033 A1 von Schaap A.P.) bekannt. Diese hydrophoben polymeren Additive verdrängen das Wasser aus der unmittelbaren Umgebung der im angeregten Zustand anfallenden Produkte, stabilisieren diese in einem hydrophoben Milieu, und erhöhen so die Photonenausbeute.

Die Verstärkung der Chemilumineszenz von Acridiniumverbindungen kann durch Verkapselung von zusätzlich hydrophobisierten Acridiniumester-Molekülen in Liposomen aus Dipalmitoylphospholipiden und Cholesterin erfolgen, um eine Akkumulierung zu erreichen (Law S-J. et al., Journal of Bioluminescence and Chemiluminescence 4, 88-98, 1989), oder aber durch Zugabe von quaternären Phosphonium-Salzen (EP 0 534 380 A1), oder Micellen aus Cetyltrimethylammoniumbromid, um eine Signalverstärkung zu erreichen (McCapra F., Accounts of Chemical Research 9, 201-208, 1976). Bezüglich des Wirkungsmechanismus wird hier eine Bevorzugung der Lichtreaktion gegenüber der Dunkelreaktion diskutiert, d.h. eine Erhöhung der Ausbeute an chemischem, in angeregtem Zustand befindlichen und somit lumineszenzfähigem Produkt.

Ein Nachteil der obengenannten Verstärkungsmaßnahmen ist, daß sie jeweils spezifisch für ein bestimmtes Lumineszenzsystem sind. Die der vorliegenden Erfindung zugrundeliegende Aufgabe bestand somit darin, eine vom jeweils verwendeten Lumineszenzsystem unabhängige und gegebenenfalls in Kombination mit anderen Verstärkungsmaßnahmen anwendbare Methode bereitzustellen, die zu einer Verbesserung der Signaldetektion bei Lumineszenzreaktionen führt.

Erfindungsgemäß wird diese Aufgabe gelöst durch ein Verfahren zum Nachweis eines Analyten in einer Probeflüssigkeit durch Lumineszenzmessung nach dem Prinzip des Ligand-Rezeptor-Assay, wobei man eine Probeflüssigkeit mit mindestens einem Rezeptor inkubiert, der eine Lumineszenzmarkierung trägt, und das Vorhandensein oder/und die Menge des nachzuweisenden Analyten in der Probeflüssigkeit durch Lumineszenzmessung bestimmt, welches dadurch gekennzeichnet ist, daß die Lumineszenzmessung in einem Meßmedium mit dispergierten Komponenten erfolgt, die eine Randomisierung des bei der Lumineszenzreaktion erzeugten Lichts und gegebenenfalls die Ausbildung einer Vorzugsrichtung bei der Lichtstreuung bewirken.

Diese durch das Vorhandensein der dispergierten Komponenten bewirkte Randomisierung bzw. Reflexion des bei der Lumineszenzreaktion erzeugten Lichts führt überraschenderweise zu einer erheblichen Steigerung der Empfindlichkeit und der Präzision der Lumineszenzmessung. Weiterhin kann z.B. bei Immobilisierung des lumineszenzmarkierten Rezeptors an eine Festphase eine bevorzugte Richtung der Lichtstreuung aufgrund der Schichtdickenverhältnisse ober- und unterhalb der Lichtquelle erreicht werden. Diese Effekte sind vom jeweils verwendeten Lumineszenzsystem unabhängig und können daher eine breite Anwendung finden. Ein weiterer Vorteil des erfindungsgemäßen Verfahrens besteht darin, daß es einfach und kostengünstig durchgeführt werden kann.

Das Meßmedium, in der beim erfindungsgemäßen Verfahren die Lumineszenzmessung erfolgt, umfaßt im allgemeinen eine Flüssigphase mit darin dispergierten, gasförmigen, flüssigen oder/und festen Komponenten. Die Dispersion besitzt günstigerweise eine ausreichende Stabilität, so daß während des Meßvorgangs keine signifikante Entmischung der dispergierten Komponenten, z.B. durch Phasentrennung oder Sedimentation, stattfindet. Bei Durchführung des erfindungsgemäßen Verfahrens in einem automatischen Meßgerät ist es bevorzugt, daß die Dispersion für mindestens einen Tag, besonders bevorzugt für mindestens 1 Woche und am meisten bevorzugt für mindestens 3 Wochen stabil ist. Beispiele für solche Dispersionen werden im folgenden angegeben.

In einer bevorzugten Ausführungsform umfaßt das Meßmedium eine Suspension oder eine kolloidale Lösung (Sol) von festen Partikeln, die günstigerweise einen mittleren Durchmesser von 10 nm bis 3 $\mu$m aufweisen. Besonders bevorzugt weisen die festen Partikel einen mittleren Durchmesser von 100 nm bis 800 nm auf. Am meisten bevorzugt besitzen die festen Partikel einen mittleren Durchmesser von 150 nm bis 600 nm. Der Anteil der festen Partikel während der Messung beträgt vorzugsweise 0,01 -2,5 % (Masse/Vol.) bezogen auf das Meßmedium und besonders bevorzugt 0,05 - 1,5% (Masse/Vol.).

Aus Gründen der Dispersionsstabilität ist es bevorzugt, daß feste dispergierte Partikel sich in ihrem spezifischen Gewicht nicht signifikant, vorzugsweise nicht mehr als 25%, und besonders bevorzugt nicht mehr als 10% vom spezifischen Gewicht des Meßmediums unterscheiden. Beispiele hierfür sind etwa Dispersionen von organischen Polymerpartikeln, z.B. gegebenenfalls funktionalisierte Acrylpolymere, Styrolpolymere, z.B. Sulfat-Latices, Amidin-Latices, Zwitterion-Latices. Weitere Beispiele sind Ethylen-, Propylen-, Butadien-, Vinyl- und Urethanpolymere sowie Copolymere der zuvor genannten Polymere. Spezifische Beispiele sind in der folgenden Tabelle dargestellt:

| Polymer | spezifische Dichte (g/cm$^3$) |
|---|---|
| Polymethylmethacrylat | 1,19 |
| Polystyrol | 1,05 |
| Polyvinyltoluol | 1,027 |
| Styrol/Butadien 95/5 (%w) | 1,05 |
| Styrol/Butadien 60/40 (%w) | 0,99 |
| Vinyltoluol/t.-Butylstyrol 63/37 (%w) | 1,00 |

Bei der Auswahl der dispergierten Partikel ist weiterhin zu beachten, daß eventuell auf den Partikeln vorhandene reaktive Gruppen mit dem jeweils verwendeten Lumineszenzsystem kompatibel sind.

In einer anderen bevorzugten Ausführungsform der vorliegenden Erfindung umfaßt das Meßmedium eine Emulsion oder kolloidale Lösung von flüssigen Partikeln mit einem mittleren Durchmesser von günstigerweise 10 nm bis 3 $\mu$m, besonders bevorzugt von 100 nm bis 1500 nm. Diese flüssigen Partikel liegen während der Messung in einem Anteil von vorzugsweise 0,01 - 3,5% (Masse/Vol.) bezogen auf das Meßmedium, besonders bevorzugt in einem Anteil von 0,1 - 1,5% (Masse/Vol.). Beispiele für stabile Dispersionen mit flüssigen Partikeln sind Lipidemulsionen in Wasser, z.B. homogenisierte Milch oder eine Emulsion von Sojalipiden oder micellierende Substanzen.

Für das erfindungsgemäße Verfahren sind grundsätzlich alle bekannten Lumineszenzmarkierungen, z.B. bio- oder chemilumineszente Direktmarkierungen, indirekte enzymverstärkte Markierungen oder Elektrochemilumineszenzmarkierungen geeignet. Ein Beispiel für bevorzugte Lumineszenzmarkierungen sind Ca-aktivierbare Photoproteine, wie Aequorin, Obelin, Clytin, Mitrocomin, Berovin und Mnemiopsin. Aequorin ist ein Ca-aktivierbares biolumineszierendes Protein aus dem Organismus Aequorea victoria. Die rekombinante Herstellung von Aequorin ist von Stults et al. (Biochemistry 31 (1992), 1433-1442) beschrieben. Die Isolierung und Reinigung des Photoproteins Obelin aus dem Organismus Obelia longissima ist von Vysotskii et al. (in Biokhimiya 54 (1989), 965-973) beschrieben. Die Klonierung, Expression und Sequenz von Obelin sind von Illarionov et al. (J. Bioluminescence and Chemiluminescence 8 (1993), VII. International Symposium-Abstracts, 88) beschrieben. Die Klonierung, Expression und Sequenzanalyse der Photoproteine Clytin und Mitrocomin sind bei Inouye und Tsuji (FEBS 315 (1993), 343-346) und Fagan et al. (FEBS 333 (1993), 301-305) beschrieben. Weitere Mitglieder der Aequorinfamilie sind die Photoproteine Berovin und Mnemiopsin (Ward und Seliger, Biochemistry 13 (1974), 1491-1499 und 1500-1510).

Die Bindung von Ca$^{2+}$ an die obengenannten Photoproteine bewirkt eine Konformationsänderung, durch die das Protein in ein Enzym überführt wird, welches eine Oxidation unter Emission von Licht katalysiert.

Weitere für die vorliegende Erfindung geeignete Lumineszenzmarkierungen sind Isoluminol oder Acridiniumverbindungen, z.B. Arylester, N-funktionalisierte Salze, Benzacridiniumverbindungen oder Carboxamide. Bei Isoluminol wird die Lumineszenzreaktion durch $H_2O_2/OH^-$ und einen Katalysator wie etwa Mikroperoxidase oder eine modifizierte prosthetische Gruppe dieses Katalysators (z.B. Deuteroferrihäm) getriggert. Acridiniumverbindungen werden durch sequentielle Zugabe von $H_2O_2/H^+$ und $OH^-$ getriggert. Die Durchführung von Lumineszenz-Immunoassays mit Isoluminol und Acridiniumverbindungen ist beispielsweise in Analytical Applications of Bioluminescence and Chemiluminescence (1984), Academic Press, Inc., insbesondere pp 149-158 und 159-162, beschrieben. Auf diese Offenbarung wird hiermit Bezug genommen.

Auch Elektrochemilumineszenzmarkierungen, z.B. lumineszierende Metallchelate, sind für das erfindungsgemäße Verfahren geeignet. Lumineszierende Metallchelate sind Metallchelate, die eine nachweisbare Elektrochemilumineszenzreaktion erzeugen. Das Metall dieser Metallchelate ist beispielweise ein Übergangsmetall oder ein Seltenerdenmetall. Vorzugsweise ist das Metall Ruthenium, Osmium, Rhenium, Iridium, Rhodium, Platin, Indium, Palladium, Molybdän, Techneticum, Kupfer, Chrom oder Wolfram. Besonders bevorzugt sind Ruthenium, Rhenium, Osmium, Chrom und Iridium. Am meisten bevorzugt ist Ruthenium.

Die Liganden, die zusammen mit dem Metall das Metallchelat bilden, sind üblicherweise aromatische N-haltige Polyheterozyklen wie etwa z.B. ggf. substituierte Bipyridine, Bipyrazole, Terpyridine und Phenanthroline. Besonders bevorzugt sind Bipyridine und Phenanthroline. Spezifische Beispiele geeigneter Metallchelate und die Durchführung von Elektrochemilumineszenzassays sind in EP-A-0 178 450, EP-A-0 580 979, WO 90/05301, WO 90/11511 und WO 92/14138 beschrieben.

Beim erfindungsgemäßen Verfahren können die dispergierten Komponenten zusammen mit dem Lumineszenztrigger, d.h. mit der die Lumineszenzreaktion auslösenden Substanz, dem Meßmedium zugesetzt werden. Andererseits können die dispergierten Komponenten auch separat vor dem Trigger zugesetzt werden.

Beim erfindungsgemäßen Verfahren wird ein zum Nachweis des jeweiligen Analyten geeigneter Rezeptor eingesetzt, der eine Lumineszenzmarkierung trägt. Diese Lumineszenzmarkierung kann eine direkt lumineszenzfähige Gruppe oder aber auch ein Enzym sein, das eine Lumineszenzreaktion katalysiert, z.B. Peroxidase oder alkalische Phosphatase. Vorzugsweise ist die Lumineszenzmarkierung eine direkt lumineszenzfähige Gruppe.

Der Rezeptor, der die Lumineszenzmarkierung trägt, kann je nach Testformat z.B. ein Antikörper oder Antikörperfragment, ein Antigen oder ein Teil davon (z.B. ein Epitop) oder ein Hapten, das gegebenenfalls an ein Trägermaterial gekoppelt ist, oder ein Partner eines spezifischen Bindepaares (z.B. Biotin/Streptavidin) oder eine Nucleinsäure sein. Verfahren zur Kopplung von lumineszenten Markierungen an Rezeptoren sind aus dem Stand der Technik bekannt und brauchen nicht ausführlich diskutiert werden.

Das erfindungsgemäße Verfahren wird nach dem Prinzip des Ligand-Rezeptor-Assay durchgeführt, d.h. ein in der Probeflüssigkeit befindlicher Analyt wird qualitativ oder/und quantitativ durch Bindung an einen markierten Rezeptor nachgewiesen. Der Nachweis des Analyten kann beispielsweise in einem Immunoassay erfolgen, der auf der spezifischen Bindung eines Antigens mit einem Antikörper beruht. Andererseits kann der Nachweis des Analyten auch in einem Hybridisierungsassay erfolgen, bei dem eine in der Probeflüssigkeit befindliche Nucleinsäure durch Hybridisierung mit einer markierten Rezeptor-Nucleinsäuresonde nachgewiesen wird. Auch eine Kombination von Immunoassay mit Hybridisierungsassay ist möglich, z.B. durch Kopplung des Nucleinsäurerezeptors mit einem Hapten, das von einem lumineszenzmarkierten Antikörper erkannt wird.

Das erfindungsgemäße Verfahren kann nach jedem beliebigen Testformat für Ligand-Rezeptor-Assays durchgeführt werden, z.B. als homogener Assay mit nur einer Reaktionsphase oder als heterogener Assay mit mehreren Reaktionsphasen, wobei die Durchführung des erfindungsgemäßen Verfahrens als heterogener Hybridisierungs- oder Immunoassay oder einer Kombination davon bevorzugt ist. Bei einem derartigen heterogenen Assay erfolgt die Inkubation des lumineszenzmarkierten Rezeptors mit einer Probeflüssigkeit in Gegenwart von mindestens einer reaktiven Festphase. Die Messung der Lumineszenzmarkierung kann gegebenenfalls nach Phasentrennung in der Flüssigphase oder/und an der Festphase erfolgen. Vorzugsweise wird die Messung der Lumineszenzmarkierung an der Festphase durchgeführt.

Bei der Durchführung des erfindungsgemäßen Verfahrens, insbesondere wenn die Messung der Lumineszenzmarkierung an einer Festphase erfolgt, ist es bevorzugt, wenn die Lumineszenzmessung durch Detektionsmodule erfolgt, die seitlich oder/und unterhalb des Meßgefäßes angebracht sind, in dem die lichterzeugende Reaktion abläuft. Bei einer derartigen Verfahrensführung sind die Vorteile der erfindungsgemäßen Zugabe dispergierter Komponenten am größten, da die Lumineszenzmarkierung an der Festphase, z.B. an der Wand des Meßgefäßes, stets in einem relativ konstanten Abstand zur Trägerwand lokalisiert ist, der typischerweise 3 - 250 nm ist.

Dieser Abstand ist zu gering für die Ausbildung einer lichtdichten Schicht, hierzu wäre eine Schichtdicke in der Dimension > 1 μm notwendig. Wird die Lumineszenzmessung erfindungsgemäß in Gegenwart dispergierter Komponenten, die beispielsweise am Ende der Immunreaktion zusammen mit dem Trigger zugegeben werden, durchgeführt, bildet sich bei Zugabe eines genügend großen Volumens eine weiße reflektierende Decke oberhalb der Lichtquelle aus, welche aufgrund der dünnen Schicht zwischen der Gefäßwand und der Lichtquelle für eine Vorzugsrichtung bei der Lichtstreuung sorgt, nämlich seitlich oder/und nach unten.

4

Dadurch wird mehr Licht auf ein seitlich oder/und unterhalb des Meßgefäßes lokalisiertes Detektionsmodul, z.B. die photosensitive Kathode einer Photomultiplier-Röhre, gelenkt, so daß die Ausbeute bei der Signaldetektion steigt.

Der Zugewinn an Sensitivität und Präzision durch die Randomisierung des bei der Lumineszenzreaktion erzeugten Lichts findet man sowohl bei Direktmarkierungen wie auch bei enzymverstärkten Markierungen, insbesondere bei angeregten Produkten mit kurzer Relaxationszeit und somit geringer diffusionsbedingter Distanzstreuung.

Bei der Durchführung des erfindungsgemäßen Verfahrens ist die Verwendung von Mikromeßgefäßen bevorzugt, die eine Bestimmung in einem Volumen von 5-1000 µl und vorzugsweise 10 - 200 µl Meßmedium erlauben.

Weiterhin betrifft die vorliegende Erfindung die Verwendung einer Dispersion, die gasförmige, flüssige oder/und feste Komponenten in einer Flüssigkeit dispergiert enthält, in einem Lumineszenz-Ligand-Rezeptor-Assay zur Randomisierung des bei der Lumineszenzreaktion erzeugten Lichts und gegebenenfalls zur Ausbildung einer Vorzugsrichtung bei der Lichtstreuung. Vorzugsweise ist die Dispersion innerhalb eines Zeitraums von mindestens einem Tag, besonders bevorzugt von mindestens einer Woche und insbesondere von mindestens 3 Wochen stabil. Eine solche stabile Dispersion kann vorteilhaft in einem automatisierten Nachweisverfahren eingesetzt werden.

Weiterhin kann die Dispersion auch die zum Triggern bzw. Aktivieren der Lumineszenzreaktion erforderlichen Komponenten enthalten. Bei Verwendung eines Ca-aktivierbaren Photoproteins als Lumineszenzmarkierung enthält die Dispersion vorzugsweise 20 bis 200 mmol/l $Ca^{2+}$-Ionen.

Außerdem kann die Dispersion gegebenenfalls Puffersubstanzen, Konservierungsmittel oder/und Stabilisatoren enthalten.

Noch ein weiterer Gegenstand der vorliegenden Erfindung ist ein Reagenz zum Nachweis eines Analyten in einer Probeflüssigkeit nach dem Prinzip des Ligand-Rezeptor-Assay, dadurch gekennzeichnet, daß es eine Dispersion von gasförmigen, flüssigen oder/und festen Komponenten in einer Flüssigkeit enthält, die eine Randomisierung des bei der Lumineszenzreaktion erzeugten Lichts und gegebenenfalls die Ausbildung einer Vorzugsrichtung bei der Lichtstreuung bewirken. Vorzugsweise ist das Reagenz innerhalb eines Zeitraums von mindestens einen Tag stabil. Außerdem kann das Reagenz weiterhin die zum Triggern der Lumineszenzreaktion erforderlichen Komponenten enthalten.

Schließlich betrifft die Erfindung auch einen Reagenzienkit zum Nachweis eines Analyten in einer Probeflüssigkeit nach dem Prinzip des Ligand-Rezeptor-Assay, umfassend ein erfindungsgemäßes Reagenz und räumlich getrennt davon einen Rezeptor, der eine Lumineszenzmarkierung trägt.

Weiterhin soll die Erfindung durch die folgenden Beispiele näher erläutert werden.

BEISPIELE

In einem ersten Set von Versuchen (Beispiele 1-5) wurden Verdünnungsreihen von rekombinantem Aequorin (Aqua-Lite™, Fa. SeaLite Sciences Inc.) hergestellt und mit einem Luminometer vermessen. Dieser Luminometer war speziell ausgelegt für eine hochsensitive Detektion von schnellen Blitzkinetiken. Die Detektion erfolgte von unten.

Hierzu wurden in miniaturisierten Reaktionsgefäßen jeweils 5 µl einer gepufferten Aequorinlösung vorgelegt und durch Injektion von 40 µl $Ca^{2+}$-Trigger die Biolumineszenzreaktion ausgelöst. Die Meßzeit betrug wahlweise 1 oder 2 Sekunden. Als $Ca^{2+}$-Trigger wurden vergleichend normale gepufferte Lösungen (100 mmol/l $CaCl_2$ in 10 mmol/l Tris-HCl, pH 7,5) oder spezielle weiße, gegebenenfalls stabilisierte Dispersionen eingesetzt.

Weiterhin wurden in den Beispielen 6 bis 8 beide Trigger-Varianten zur Quantifizierung von TSH- und Digoxigenin-Eichreihen mittels entsprechender 1-Schritt Biolumineszenz-Immunoassays vergleichend bewertet, bei denen sowohl das eingesetzte Reaktionsvolumen als auch die Inkubationszeit gegenüber dem gegenwärtigen Stand der Technik deutlich reduziert wurden, aber dennoch eine hohe Testsensitivität erreicht werden soll, was höchste Ansprüche an die Detektionsempfindlichkeit stellt.

Untersucht wurde sowohl der Zugewinn an detektiertem Signal als auch der Zugewinn an Präzision und schließlich die Auswirkung auf die erreichbare Testsensitivität.

Abkürzungen:

| S- / A-Latex | = Sulfat- bzw. Amidin-Latex |
|---|---|
| RLU | = Relative light units |
| RLU/sec | = Relative light units pro Sekunde |
| cps | = Impulse pro Sekunde |
| SD | = Standard Deviation = Standardabweichung (mit n-1-Gewichtung) |

$$VK = \text{Variationskoeffizient} = \frac{SD}{\bar{x}} \cdot 100$$

| $\bar{x}$ | = Mittelwert mehrerer Messungen |
|---|---|
| Intralipid | = Emulsion von Sojabohnenöl, Phosphatidylcholin und Glycerin in Wasser (eingetragenes Warenzeichen der Fa. Pfrimmer Kabi GmbH & Co.KG). |

Beispiel 1a:

Aequorin-Labelsensitivität von Triggermedien, die eine Lipidemulsion enthalten

Triggermedien:

a. 100 mmol/l $CaCl_2$ in 10 mmol/l Tris, pH 7,5
b. homogenisierte Milch, 3,5% Fett, 1:20-Verdünnung in Tris/$CaCl_2$

Resultate: Angaben in [RLU/sec]

| Aequorin **mol/5 μl** | **Trigger a** | **Trigger b** |
|---|---|---|
| $9 \times 10^{-16}$ | 119.550 | 165.665 |
| $9 \times 10^{-17}$ | 12.640 | 15.520 |
| $9 \times 10^{-18}$ | 1.300 | 2.120 |
| $9 \times 10^{-19}$ | 130 | 185 |
| $9 \times 10^{-20}$ | 22 | 37 |
| Leerwert | 12 | -- |
| **F =** | **1,00** | **1,42** |

F ist der mittlere Wirkungsquotient im Bereich $10^{-16}$ bis $10^{-19}$ mol. Der Wirkungsquotient des Standard-Triggers a wurde als Referenz verwendet. Es wurde gefunden, daß die Triggermedien, die eine Lipidemulsion enthalten, einen besseren mittleren Wirkungsquotienten als der Standard-Trigger zeigen.

Beispiel 1b:

Aequorin-Labelsensitivität von Triggermedien, die eine Lipidemulsion enthalten

Vergleich: klare $CaCl_2$-Lösung gegen weiße Intralipid-Emulsion (0,48 %) in Tris/$CaCl_2$
Ergebnisse in [RLU/sec], 0,2-1,2 sec nach Start

| Aequorin [mol/5 μl] | klarer Trigger (Lösung) | weißer Trigger (Emulsion) |
|---|---|---|
| $1 \times 10^{-14}$ | 1.430.116 | 2.081.756 |
| $1 \times 10^{-15}$ | 152.863 | 283.489 |
| $1 \times 10^{-16}$ | 15.659 | 26.887 |
| $1 \times 10^{-17}$ | 1.517 | 2.678 |
| $1 \times 10^{-18}$ | 217 | 268 |
| $1 \times 10^{-19}$ | 49 | 105 |
| $1 \times 10^{-20}$ | 4 | 23 |
| Pufferleerwert | 2 | 6 |
| F | 1,00 | 1,70 |
| F = mittlerer Wirkungsquotient im Bereich $10^{-14}$-$10^{-19}$ mol | | |

Beispiel 2:

Aequorin-Labelsensitivität von Triggermedien, die eine Latexsuspension enthalten

Triggermedien:

a. 100 mmol/l $CaCl_2$ in 10 mmol/l Tris, pH 7,5
b. 100 mmol/l $CaCl_2$ in 0.5% Suspension aus Amidin-Latex (299 nm Partikeldurchmesser)
c. 100 mmol/l $CaCl_2$ in 0.5% Suspension aus Zwitterion-Latex (238 nm Partikeldurchmesser)

Beide Latices waren in 1 mmol/l Glycinpuffer, pH 7,4, mit 0,1 % Tween-20 suspendiert.

Resultate: Angaben in [RLU / 2 sec],
Mittelwerte aus Quadruplikatmessungen

| Aequorin **mol/5 $\mu$l** | **Trigger a** | **Trigger b** | **Trigger c** |
|---|---|---|---|
| $10^{-15}$ | 216.750 | 273.618 | 246.865 |
| $10^{-16}$ | 22.670 | 29.169 | 27.108 |
| $10^{-17}$ | 1.935 | 2.766 | 2.537 |
| $10^{-18}$ | 186 | 317 | 274 |
| $10^{-19}$ | 30 | 47 | 32 |
| $10^{-20}$ | 23 | 23 | 8 |
| Leerwert | 17 | 15 | 23 |
| **r =** | **1,00** | **1,00** | **1,00** |
| **F =** | **1,00** | **1,45** | **1,24** |

r = Regressionsanalyse im Meßbereich von $10^{-15}$ bis $10^{-19}$ mol. Referenz ist die Standard-Triggerlösung a.
F = mittlerer Wirkungsquotient im Bereich $10^{-15}$ bis $10^{-19}$ mol.

Beispiel 3:

Einfluß des Feststoffanteils auf die Aequorin-Labelsensitivität

Triggermedien:

a. 100 mmol/l $CaCl_2$ in 10 mmol/l Tris, pH 7,5
b. 100 mmol/l $CaCl_2$ in 0,5 % Suspension aus Amidin-Latex (299 nm Partikeldurchmesser)
c. 100 mmol/l $CaCl_2$ in 1,0 % Suspension aus Amidin-Latex (299 nm Partikeldurchmesser)

Beide Latices waren in 1 mmol/l Glycinpuffer, pH 7,4, mit 0,1% Tween-20 suspendiert.

Resultate: Angaben in [RLU / 2 sec]
Mittelwerte aus Quadruplikatmessungen

| Aequorin mol/5 $\mu$l | Trigger a | Trigger b | Trigger c |
|---|---|---|---|
| $10^{-15}$ | 230.061 | 317.802 | 297.537 |
| $10^{-16}$ | 23.186 | 33.989 | 31.570 |
| $10^{-17}$ | 2.013 | 2.889 | 2.856 |
| $10^{-18}$ | 205 | 306 | 324 |
| $10^{-19}$ | 30 | 45 | 44 |
| $10^{-20}$ | 8 | 37 | 13 |
| Leerwert | 25 | 16 | 23 |
| **r =** | **1,00** | **1,00** | **1,00** |
| **F =** | **1,00** | **1,46** | **1,42** |

r = Regressionsanalyse im Meßbereich von $10^{-15}$ bis $10^{-19}$ mol. Referenz ist die Standard-Triggerlösung a.
F = mittlerer Wirkungsquotient im Bereich $10^{-15}$ bis $10^{-19}$ mol.

Beispiel 4:

Einfluß der Partikelgröße ($\leq$ 299 nm) auf die Aequorin-Labelsensitivität

Triggermedien:

a. 100 mmol/l $CaCl_2$ in 10 mmol/l Tris, pH 7,5
b. 100 mmol/l $CaCl_2$ in 0,1% Suspension aus Amidin-Latex (116 nm Partikeldurchmesser)
c. 100 mmol/l $CaCl_2$ in 0,1% Suspension aus Amidin-Latex (299 nm Partikeldurchmesser)
d. 100 mmol/l $CaCl_2$ in 0,1% Suspension aus Zwitterion-Latex (238 nm Partikeldurchmesser)

Alle Latices waren in 1 mmol/l Glycinpuffer, pH 7,4, mit 0,1% Tween-20 suspendiert.

Resultate: Angaben in [RLU / 2 sec]

| Aequorin mol/5 $\mu$l | Trigger a | Trigger b | Trigger c | Trigger d |
|---|---|---|---|---|
| $10^{-15}$ | 246.869 | 241.232 | 271.882 | 254.887 |
| $10^{-16}$ | 23.823 | 26.386 | 28.875 | 26.712 |
| $10^{-17}$ | 2.272 | 2.346 | 2.713 | 2.677 |
| $10^{-18}$ | 224 | 236 | 240 | 271 |
| $10^{-19}$ | 25 | 29 | 32 | 52 |
| $10^{-20}$ | 8 | 21 | 10 | 12 |
| Leerwert | 5 | 16 | 16 | 15 |
| r = | 1,00 | 1,00 | 1,00 | 1,00 |
| F = | 1,00 | 1,07 | 1,17 | 1,32 |

r = Regressionsanalyse im Meßbereich von $10^{-15}$ bis $10^{-19}$ mol. Referenz ist die Standard-Triggerlösung a.
F = mittlerer Wirkungsquotient im Bereich $10^{-15}$ bis $19^{-19}$ mol.

Beispiel 5a:

Einfluß der Partikelgröße ($\geq$ 299 nm) auf die Aequorin-Labelsensitivität

Triggermedien:

    a. 100 mmol/l $CaCl_2$ in 10 mmol/l Tris, pH 7,5
    b. 100 mmol/l $CaCl_2$ in 0,5% Suspension aus Amidin-Latex (299 nm Partikeldurchmesser)
    c. 100 mmol/l $CaCl_2$ in 0,5% Suspension aus Amidin-Latex (480 nm Partikeldurchmesser)
    d. 100 mmol/l $CaCl_2$ in 0,5% Suspension aus Amidin-Latex (600 nm Partikeldurchmesser)

Alle Latices waren in 1 mmol/l Glycinpuffer, pH 7,4, mit 0,1% Tween-20 suspendiert. Es wurden frisch hergestellte Suspensionen verwendet.

Resultate: Angaben in [RLU / 2 sec]

| Aequorin mol/5 $\mu$l | Trigger a | Trigger b | Trigger c | Trigger d |
|---|---|---|---|---|
| $10^{-15}$ | 210.459 | 267.907 | 267.945 | 303.296 |
| $10^{-16}$ | 20.162 | 25.932 | 30.194 | 32.227 |
| $10^{-17}$ | 1.729 | 2.492 | 3.137 | 3.138 |
| $10^{-18}$ | 167 | 253 | 280 | 320 |
| $10^{-19}$ | 17 | 57 | 48 | 38 |
| $10^{-20}$ | 8 | 28 | 25 | 14 |
| Leerwert | 10 | 11 | 22 | 20 |
| **r =** | **1,00** | **0,9999** | **0,9998** | **0,9999** |
| **F =** | **1,00** | **1,77** | **1,82** | **1,80** |

r = Regressionsanalyse im Meßbereich von $10^{-15}$ bis $10^{-19}$ mol. Referenz ist die Standard-Triggerlösung a.
F = mittlerer Wirkungsquotient im Bereich $10^{-15}$ bis $19^{-19}$ mol.

Beispiel 5b:

Einfluß der Partikelgröße ($\geqq$ 299 nm) auf die Aequorin-Labelsensitivität

Triggermedien:

a. 100 mmol/l $CaCl_2$ in 10 mmol Tris, pH 7,5
b. 100 mmol/l $CaCl_2$ in 0,5% Suspension aus Amidin-Latex (299 nm Partikeldurchmesser)
c. 100 mmol/l $CaCl_2$ in 0,5% Suspension aus Amidin-Latex (480 nm Partikeldurchmesser)
d. 100 mmol/l $CaCl_2$ in 0,5% Suspension aus Amidin-Latex (600 nm Partikeldurchmesser)

Alle Latices waren in 1 mmol/l Glycinpuffer, pH 7,4, mit 0,1% Tween-20 suspendiert. Die Suspensionen waren 3 Wochen bei 4°C gelagert.

Resultate: Angaben in [RLU / 2 sec]

| Aequorin **mol/5** $\mu$l | **Trigger a** | **Trigger b** | **Trigger c** | **Trigger d** |
|---|---|---|---|---|
| $10^{-15}$ | 198.120 | 286.136 | 290.122 | 275.504 |
| $10^{-16}$ | 17.951 | 32.957 | 25.881 | 27.818 |
| $10^{-17}$ | 1.761 | 2.895 | 2.614 | 2.535 |
| $10^{-18}$ | 188 | 275 | 302 | 281 |
| $10^{-19}$ | 16 | 36 | 43 | 32 |
| $10^{-20}$ | 8 | 17 | 13 | 23 |
| Leerwert | 13 | 21 | 21 | 11 |
| **r =** | **1,00** | **0,9997** | **0,9999** | **0,9999** |
| **F =** | **1,00** | **1,73** | **1,74** | **1,57** |

r = Regressionsanalyse im Meßbereich von $10^{-15}$ bis $10^{-19}$ mol. Referenz ist die Standard-Triggerlösung a.
F = mittlerer Wirkungsquotient im Bereich $10^{-15}$ bis $19^{-19}$ mol.

Beispiel 6:

Anwendung einer weißen Trigger-Präparation in einem TSH-Biolumineszenz-Immunoassay

In miniaturisierte, Streptavidin-beschichtete Reaktionsgefäße wurde eine Mischung aus 13 $\mu$l Standardlösung und 27 $\mu$l Testpuffer gegeben. Der Testpuffer enthielt $2 \times 10^{-8}$ mol/l biotinyliertes Anti-TSH-IgG und ca. $10^6$ RLU/sec eines Anti-TSH-IgG-Aequorin-Konjugats, das durch Kopplung von Maleimid-aktiviertem Anti-TSH-Antikörper an eine durch vorherige Umsetzung mit 2-Iminothiolan eingeführte SH-Gruppe von Aequorin hergestellt worden war. Der biotinylierte und der lumineszenzmarkierte Antikörper waren jeweils gegen unterschiedliche Epitope von TSH gerichtet. Nach 15 Minuten Simultan-Inkubation bei Raumtemperatur ohne Schütteln erfolgte die Trennung von festphasengebundenem, über Immunkomplexe immobilisiertem Konjugat von ungebundenem, noch in der Flüssigphase vorliegendem Konjugat. Danach wurde die Aequorin-Biolumineszenz in einem Luminometer durch Zugabe von jeweils 100 mmol/l $Ca^{2+}$-Ionen ausgelöst:

A. einmal mit $Ca^{2+}$-Ionen gelöst in 10 mmol/l Tris-Puffer, pH 7,5 (= Normaltrigger)
B. zum anderen mit $Ca^{2+}$-Ionen gelöst in 1 mmol/l Glycin-Puffer, in welchem farblose Amidin-Latexbeads (480 nm Partikelgröße) in einer Konzentration von 0,5% Feststoffanteil suspendiert waren (= weißer Trigger)

Ergebnis: Es wurden die folgenden Eichkurven erhalten:

| Kalibrator [µU TSH/ml] | A Ca$^{2+}$ Normaltrigger [RLU/s gebunden] | B Ca$^{2+}$/weißer Trigger [RLU/s gebunden] | B/A |
|---|---|---|---|
| 0,0 µU/ml | 29 ± 8 | 36 ± 3 | 1,24 |
| 0,50 µU/ml | 177 | 289 | 1,63 |
| 4,64 µU/ml | 1483 | 2553 | 1,72 |
| 9,05 µU/ml | 3003 | 5767 | 1,92 |
| 21,81 µU/ml | 6754 | 13486 | 2,00 |
| 42,78 µU/ml | 14195 | 27075 | 1,91 |
| Dynamik [a] | 489 | 759 | |
| Sensitivität [b] | 0,054 µU TSH/ml | 0,012 µU TSH/ml | |

[a] = Quotient höchster / niedrigster Standard
[b] = berechnet aus dem Mittelwert einer Quadruplikatmessung des Nullstandards plus 2-fache Standardabweichung auf Signalbasis, über lineare Regression auf Konzentrationen umgerechnet.

Dieser Versuch demonstriert das Potential der erfindungsgemäßen Methode für erhöhte analytische Sensitivität in Biolumineszenz-Immunoassays.

Beispiel 7:

Anwendung einer weißen Trigger-Präparation in einem DIG-Biolumineszenz-Immunoassay

In miniaturisierte, Streptavidin-beschichtete Reaktionsgefäße wurde eine Mischung aus 13 µl Standardlösung eines Heptapeptids mit der Sequenz Ser-Gln-Asn-Tyr-Pro-Ile-Val, das biotinyliert und mit Digoxigenin markiert war, und 27 µl Testpuffer pipettiert, welcher ca. 2 10$^6$ RLU/s Anti-DIG-Fab-Aequorin-Konjugat enthielt.

Nach 15 Minuten Simultan-Inkubation bei Raumtemperatur ohne Schütteln erfolgte die Trennung von festphasengebundenem, über Immunkomplexe immobilisiertem Konjugat von ungebundenem, noch in der Flüssigphase vorliegendem Konjugat.

Danach wurde die Aequorin-Biolumineszenz in einem Luminometer durch Zugabe von jeweils 100 mmol/l Ca$^{2+}$-Ionen ausgelöst:

1: einmal mit Ca$^{2+}$-Ionen gelöst in 10 mmol/l Tris-Puffer, pH 7,5 (= Normaltrigger)
2: zum anderen mit Ca$^{2+}$-Ionen gelöst in 1 mmol/l Glycin-Puffer, in welchem farblose Amidin-Latexbeads (600 nm-Partikelgröße) in einer Konzentration von 0,5% Feststoffanteil suspendiert waren (= weiße Trigger)

Ergebnis: Es wurden die folgenden Eichkurven erhalten:

**DIG-Assay 1 (Normaltrigger)**

| | Einzelmessungen | Mittelwert ± SD | Faktor X/A |
|---|---|---|---|
| Kalibrator A 0 pmol/l | 83 | 92 cps ± 19 20% VK | |
| | 103 | | |
| | 112 | | |
| | 71 | | |
| Kalibrator B 0,2 pmol/l | 187 | 205 cps | 2,22 |
| | 133 | | |
| | 239 | | |
| | 259 | | |
| Kalibrator C 2 pmol/l | 1056 | 976 cps | 10,57 |
| | 895 | | |
| Kalibrator D 20 pmol/l | 8826 | 8621 cps | 93,45 |
| | 8416 | | |
| Kalibrator E 200 pmol/l | 83419 | 78384 cps | 849,69 |
| | 73349 | | |
| Kalibrator F 1000 pmol/l | 305260 | 272774 cps | 2956,89 |
| | 240287 | | |

Untere Nachweisgrenze (UNG):

$$\text{UNG } (A + 2 \cdot \text{SD, lineare Regression}) = 8,7 \cdot 10^{-19} \text{ mol DIG}$$
$$= 5,3 \cdot 10^5 \text{ DIG-Moleküle}$$

**DIG-Assay 2 (weißer Trigger)**

| | Einzelmessungen | Mittelwert ± SD | Faktor X/A |
|---|---|---|---|
| Kalibrator A 0 pmol/l | 160 | 165 cps ± 21 13% VK | |
| | 180 | | |
| | 181 | | |
| | 137 | | |
| Kalibrator B 0,2 pmol/l | 347 | 365 cps | 2,22 |
| | 356 | | |
| | 498 | | |
| | 258 | | |
| Kalibrator C 2 pmol/l | 1508 | 1332 cps | 8,09 |
| | 1155 | | |
| Kalibrator D 20 pmol/l | 11099 | 11292 cps | 68,64 |
| | 11485 | | |
| Kalibrator E 200 pmol/l | 108984 | 105553 cps | 641,66 |
| | 102122 | | |
| Kalibrator F 1000 pmol/l | 392615 | 408689 cps | 2484,43 |
| | 424763 | | |

Untere Nachweisgrenze:

$$\text{UNG (A + 2} \cdot \text{SD, lineare Regression)} = 3,4 \cdot 10^{-19} \text{ mol DIG}$$
$$= 2,0 \cdot 10^{5} \text{ DIG-Moleküle}$$

Wiederum zeigt sich das Potential der erfindungsgemäßen Methode zur Verbesserung von Signaldetektion, Präzision, und somit auch der analytischen Sensitivität.

**Beispiel 8:**

Anwendung einer weißen Trigger-Präparation in einem DIG-Biolumineszenz-Immunoassay

Der Versuchsaufbau war wie bei Beispiel 7. Es wurde besonders auf die Präzision im unteren Konzentrationsbereich geachtet. Es wurden $1,5 \cdot 10^{6}$ RLU/s eines hochgereinigten ⟨DIG⟩-Fab-Aequorin-1:1-Konjugates pro Test und Streptavidin-beschichtete Reaktionsgefäße aus einer Produktionscharge, die funktionell als sehr gut bewertet war, verwendet. Bei diesem Experiment trat der Präzisionszugewinn aufgrund von Signalverstärkung und Randomisierung besonders deutlich hervor.

Ergebnis: Es wurden die folgenden Eichkurven erhalten:

**DIG-Assay 1** (Normaltrigger)

|  | Einzelmessungen | Mittelwert ± SD | Faktor X/A |
|---|---|---|---|
| Kalibrator A 0 pmol/l | 112 | 110 cps ± 24 21 % VK | |
| | 141 | | |
| | 86 | | |
| | 99 | | |
| Kalibrator B 0,2 pmol/l | 188 | 162 cps ± 21 13 % VK | 1,48 |
| | 170 | | |
| | 145 | | |
| | 144 | | |
| Kalibrator C 2 pmol/l | 883 | 828 cps | 7,56 |
| | 772 | | |
| Kalibrator D 20 pmol/l | 8007 | 7678 cps | 70,12 |
| | 7349 | | |
| Kalibrator E 200 pmol/l | 85774 | 80096 cps | 731,47 |
| | 74418 | | |
| Kalibrator F 1000 pmol/l | 249480 | 247803 cps | 2263,04 |
| | 246125 | | |

Untere Nachweisgrune (UNG)

$$\text{UNG (A + 2} \cdot \text{SD, lineare Regression)} = 2,4 \cdot 10^{-18} \text{ mol DIG}$$
$$= 1,45 \cdot 10^{6} \text{ DIG-Moleküle}$$

<u>**DIG-Assay 2**</u> (Weißer Trigger; 0,5 % Feststoffanteil)

|  | Einzel messungen | Mittelwert ± SD | Faktor X/A |
|---|---|---|---|
| Kalibrator A 0 pmol/l | 167 | 160 cps ± 7 5 % VK | |
| | 164 | | |
| | 150 | | |
| | 159 | | |
| Kalibrator B 0,2 pmol/l | 254 | 273 cps ± 22 8 % VK | 1,70 |
| | 253 | | |
| | 294 | | |
| | 289 | | |
| Kalibrator C 2 pmol/l | 1302 | 1213 cps | 7,55 |
| | 1123 | | |
| Kalibrator D 20 pmol/l | 11789 | 11425 cps | 71,41 |
| | 11061 | | |
| Kalibrator E 200 pmol/l | 94536 | 102497 cps | 640,61 |
| | 110458 | | |
| Kalibrator F 1000 pmol/l | 424678 | 405070 cps | 2531,68 |
| | 385461 | | |

Untere Nachweisgrune (UNG)

$$\text{UNG} (A + 2 \cdot \text{SD, lineare Regression}) = 3,2 \cdot 10^{-19} \text{ mol DIG}$$
$$= 2,0 \cdot 10^5 \text{ DIG-Moleküle}$$

**Patentansprüche**

1. Verfahren zum Nachweis eines Analyten in einer Probeflüssigkeit nach dem Prinzip des Ligand-Rezeptor-Assay, wobei man die Probeflüssigkeit mit mindestens einem Rezeptor inkubiert, der eine Lumineszenzmarkierung trägt, und das Vorhandensein oder/und die Menge des nachzuweisenden Analyten in der Probeflüssigkeit durch Lumineszenzmessung bestimmt,
**dadurch gekennzeichnet**,
daß die Lumineszenzmessung in einem Meßmedium mit dispergierten Komponenten erfolgt, die eine Randomisierung des bei der Lumineszenzreaktion erzeugten Lichts und gegebenenfalls die Ausbildung einer Vorzugsrichtung bei der Lichtstreuung bewirken.

2. Verfahren nach Anspruch 1,
**dadurch gekennzeichnet**,
daß das Meßmedium eine Flüssigphase mit darin dispergierten, gasförmigen, flüssigen oder/und festen Komponenten umfaßt.

3. Verfahren nach Anspruch 1 oder 2,
**dadurch gekennzeichnet**,
daß das Meßmedium eine Suspension oder kolloidale Lösung von festen Partikeln mit einem mittleren Durchmesser von 10 nm bis 3 μm enthält.

4. Verfahren nach Anspruch 3,
**dadurch gekennzeichnet**,
daß die festen Partikel einen mittleren Durchmesser von 100 nm bis 800 nm aufweisen.

5. Verfahren nach Anspruch 3 oder 4,
**dadurch gekennzeichnet**,
daß die festen Partikel während der Messung in einem Anteil von 0,01 - 2,5 % (Masse/Vol.) bezogen auf das Meßmedium vorliegen.

6. Verfahren nach einem der Ansprüche 1-5,
**dadurch gekennzeichnet**,
daß die Lumineszenzmarkierung des Rezeptors aus bio- oder chemilumineszenten Direktmarkierungen, enzymverstärkten Markierungen oder Elektrochemilumineszenzmarkierungen ausgewählt wird.

7. Verfahren nach einem der Ansprüche 1-6,
**dadurch gekennzeichnet**,
daß die Inkubation des lumineszenzmarkierten Rezeptors mit der Probeflüssigkeit nach dem Prinzip des heterogenen Assay in Gegenwart von mindestens einer reaktiven Festphase erfolgt.

8. Verfahren nach einem der Ansprüche 1-7,
**dadurch gekennzeichnet**,
daß die Lumineszenzmessung durch Detektionsmodule erfolgt, die seitlich oder/und unterhalb des Reaktionsgefäßes vorgesehen sind.

9. Verfahren nach einem der Ansprüche 1 bis 8,
**dadurch gekennzeichnet**,
daß der Nachweis des Analyten in einem Immunoassay, einem Hybridisierungsassay oder einer Kombination davon erfolgt.

10. Verwendung einer Dispersion, die gasförmige, flüssige oder/und feste Komponenten in einer Flüssigkeit dispergiert enthält, in einem Lumineszenz-Ligand-Rezeptor-Assay zur Randomisierung des bei der Lumineszenzreaktion erzeugten Lichts und gegebenenfalls zur Ausbildung einer Vorzugsrichtung bei der Lichtstreuung.

11. Reagenz zum Nachweis eines Analyten in einer Probeflüssigkeit nach dem Prinzip des Ligand-Rezeptor-Assay,
**dadurch gekennzeichnet**,
daß es eine Dispersion von gasförmigen, flüssigen oder/und festen Komponenten in einer Flüssigkeit enthält, die eine Randomisierung des bei einer Lumineszenzreaktion erzeugten Lichts und gegebenenfalls die Ausbildung einer Vorzugsrichtung bei der Lichtstreuung bewirken.

12. Reagenzienkit zum Nachweis eines Analyten in einer Probeflüssigkeit nach dem Prinzip des Ligand-Rezeptor-Assay, umfassend ein Reagenz nach Anspruch 11 und räumlich getrennt davon einen Rezeptor, der eine Lumines-zenzmarkierung trägt.